# FASCICULE DE BREVET EUROPEEN

(11) **EP 0 740 928 B1**
(45) Date de publication et mention de la délivrance du brevet: **07.07.2004**
(21) Numéro de dépôt: 96200816.5
(22) Date de dépôt: 27.03.1996
(51) Int. Cl.: A61F 2/06

(54) **Tuteur auto-expansible pour dispositif médical à introduire dans une cavité d'un corps, et son procédé de préparation**
Selbstexpandierender Stent zur Einführung einer medizinischen Vorrichtung in eine Körperhöhle und Herstellungsverfahren
Self-expanding stent for introducing a medical device in a body cavity and manufacturing process

(30) Priorité: 12.04.1995 BE 9500334; 12.04.1995 BE 9500335
(43) Date de publication de la demande: 06.11.1996
(73) Titulaire: CORVITA EUROPE, 1070 Bruxelles (BE)
(72) Inventeur: Frid, Noureddine, 1650 Beersel (BE)
(74) Mandataire: Claeys, Pierre

(56) Documents cités:
- EP-A- 0 183 372
- EP-A- 0 615 769
- EP-A- 0 621 015
- EP-A- 0 659 389
- WO-A-91/12779
- WO-A-95/09586
- GB-A- 1 205 743
- US-A- 5 061 275
- US-A- 5 073 315
- US-A- 5 476 508

## Description

La présente invention est relative à un tuteur auto-expansible pour dispositif médical à introduire dans une cavité d'un corps humain ou animal, comprenant un corps tubulaire radialement expansible et rétractable entre un premier diamètre, correspondant à un état de travail du tuteur, et un deuxième diamètre supérieur au premier, correspondant à un état de repos du tuteur, et axialement expansible et rétractable entre une première longueur, correspondant à l'état de travail, et une deuxième longueur inférieure à la première, correspondant à l'état de repos, ce corps tubulaire comportant des premiers fils rigides flexibles qui sont disposés côte à côte au nombre d'au moins deux en formant des premiers fils multiples enroulés suivant un premier sens hélicoïdal autour d'un axe longitudinal du corps tubulaire, et des deuxièmes fils rigides flexibles qui sont disposés côte à côte au nombre d'au moins deux en formant des deuxièmes fils multiples enroulés suivant un deuxième sens hélicoïdal inverse au premier autour de l'axe longitudinal susdit, chaque fil multiple enroulé dans un desdits sens croisant des fils multiples enroulés dans l'autre sens suivant une disposition tressée.

On connaît depuis longtemps des tuteurs qui sont utilisés comme dilatateurs vasculaires, oesophagiens ou autres (GB-1205743). Ces tuteurs formés d'une tresse tubulaire de fils individuels comportent, à l'état de repos, des fils formant un angle assez petit par rapport à une génératrice du tube, c'est-à-dire d'au maximum 30°. Les tuteurs de ce genre n'offrent pas une capacité d'expansion radiale suffisante lorsqu'ils sont libérés après avoir été radialement comprimés. Leur résistance à l'écrasement est également insuffisante lors de leur utilisation dans des cavités du corps dont les parois exercent une forte pression radiale sur le tuteur introduit.

On a par conséquent cherché à remédier à ces inconvénients en prévoyant un tressage des fils individuels suivant un angle plus grand, de façon que, à l'état de repos du tuteur, les fils présentent un angle supérieur à 45°, de préférence d'au moins 60° par rapport à une génératrice du tuteur (US-A-4655711 et US-A-4954126). Toutefois, les tuteurs à grand angle de tressage présentent un grand inconvénient. En effet, le tuteur tubulaire doit être allongé de 2 à 3 fois sa longueur initiale pour pouvoir être inséré dans un introducteur vasculaire. Il occupe alors de 40 à 50 % de la longueur de ce dernier, en le rigidifiant et en rendant compliqué son passage à travers la voie fémorale par laquelle l'introduction dans le corps est généralement amorcée. Lors du largage, la structure tubulaire se rétracte de deux à trois fois en longueur. Il est dès lors très difficile d'estimer la longueur qui va se déployer dans la cavité interne du corps traité et l'endroit précis où le tuteur va s'ancrer, ce qui peut entraîner de sérieux problèmes. Par exemple, une longueur de tuteur peut se déployer au-delà d'une bifurcation vasculaire et entraîner ainsi une fermeture d'artère non souhaitable, lorsque le tuteur est recouvert d'un revêtement, comme c'est le cas pour les endoprothèses luminales. Dans un autre cas, si la longueur déployée est trop courte, l'anévrisme traité ne sera pas fermé. Enfin, il est apparu que les tuteurs à grand angle de tressage et à fils individuels ne présentaient pas une bonne résistance à la pression artérielle, à l'intérieur des anévrismes traités. A l'intérieur de l'anévrisme, c'est-à-dire là où les tuteurs ne subissent plus de compression radiale de la part de la paroi vasculaire, ces derniers ont tendance à ballonner, ce qui favorise des pertes d'étanchéité.

D'autres tresses tubulaires à base de fils, qui sont utilisées dans des dispositifs à introduire dans le corps humain, sont aussi décrites notamment dans EP-A-0183372, US-A-3509883, US-A-5061275 et US-A-5171262.

Dans le brevet US-A-5061275, en particulier, on a prévu la possibilité de façonner des corps tubulaires tressés dont les extrémités s'évasent de manière conique par rapport au reste du corps qui est de forme cylindrique. Sans résoudre le problème du ballonnement précité dans les anévrismes, cette forme de réalisation du tuteur ne permet que peu d'adaptation de la longueur au cas à traiter. Dès que le chirurgien découpe une longueur trop importante d'une extrémité par exemple d'une endoprothèse munie d'un tel tuteur, cette extrémité ne montre plus d'évasement et a perdu l'avantage que lui conférait ce dernier.

On connaît enfin des tuteurs en forme de tresses tubulaires expansibles pour des prothèses destinées à fournir des médicaments à l'intérieur du système vasculaire (WO 91/12779). Suivant une forme de réalisation décrite dans ce document, les filaments tressés se présentent sous la forme d'un fil multiple, trois filaments étant respectivement tordus ensemble en un toron pour former le fil multiple. Pour l'homme de métier moyen, il est clair qu'une telle forme de réalisation sera difficilement applicable. Elle va présenter un encombrement volumique insupportable et à l'endroit du croisement entre les torons il va se produire un frottement important. Enfin, à cause de ces torons, il va être compliqué de réduire suffisamment le diamètre du tuteur pour pouvoir l'insérer dans un introducteur. En cas de filaments métalliques, ceux-ci vont souffrir énormément et une casse n'est pas à exclure.

Lors d'essais de fabrication de tresse en fils toronnés, on a observé que, pendant le traitement thermique la structure torsadée contenait des particules qui restaient emprisonnées à l'intérieur de celle-ci, particules très difficiles à extraire, même par nettoyage aux ultrasons. Or, la propreté de la structure qui va être en contact avec le sang est d'une importance capitale, et ces particules peuvent affecter la biocompatibilité de l'endoprothèse.

Suivant l'invention on a résolu les problèmes mentionnés ci-dessus par un tuteur auto-expansible tel que décrit au début, dans lequel tous les premiers fils rigides flexibles de chaque premier fil multiple suivent un parcours parallèle et croisent tous les deuxièmes fils rigides flexibles de chaque deuxième fil multiple croisé, du même côté du deuxième fil multiple croisé, et en ce que tous les deuxièmes fils rigides flexibles de chaque deuxième fil multiple suivent un parcours parallèle et croisent tous les premiers fils rigides flexibles de chaque premier fil multiple croisé, du même côté du premier fil multiple croisé.

Un tel tuteur présente une grande stabilité dimensionnelle et géométrique, sans nécessiter de grand angle de tressage et, de ce fait, la précision au largage de ce tuteur en est fortement augmentée. Par ailleurs, on peut réduire sans problème le diamètre d'un tel tuteur à celui d'un introducteur courant, les fils multiples utilisés suivant l'invention ne présentant aucune entrave à cela.

Avantageusement tous les premiers fils susdits et tous les deuxièmes fils susdits forment des fils multiples susdits.

Par tuteur auto-expansible, il faut entendre suivant l'invention que, amené dans sa position de travail, c'est-à-dire une position radialement comprimée et axialement étendue, le tuteur, une fois libéré, tend à récupérer spontanément sa position de repos, c'est-à-dire une position radialement expansée et axialement contractée.

Dans la disposition tressée des fils, dans certaines formes de réalisation, un premier fil multiple va, lors d'un croisement avec un deuxième fil multiple, passer par-dessus celui-ci et lors du croisement suivant passer en dessous du deuxième fil alors croisé, et ainsi de suite. Dans d'autres formes de réalisation, le premier fil multiple passe au-dessus d'un deuxième fil multiple à deux croisements successifs ou davantage et passera alors seulement en dessous d'un deuxième fil multiple après plusieurs croisements successifs et ainsi de suite. Des dispositions tressées mixtes peuvent aussi être prévues, ainsi que des tresses formées partiellement de doubles fils et de monofils.

De toutes manières, par fils multiples suivant l'invention, il faut entendre que les fils rigides flexibles, qui les forment, sont disposés côte à côte et suivent un parcours parallèle dans la disposition tressée. Dès le moment où deux fils voisins ne suivent plus le même parcours, c'est-à-dire par exemple que l'un croise un fil enroulé en sens inverse en passant par-dessus tandis que l'autre croise ce même fil enroulé en sens inverse en passant en dessous, il ne s'agit plus de fils formant un fil multiple au sens de la présente invention. Les fils formant un même fil multiple peuvent être jointifs ou disposés à très faible distance l'un de l'autre.

Par dispositif médical à introduire dans une cavité d'un corps humain ou animal, il faut entendre par exemple les endoprothèses luminales, les cathéters, les dilatateurs, les greffes et analogues qui peuvent être introduits par exemple dans des cavités vasculaires, oesophagiennes, urinaires, uretérales, biliaires, et autres conduits tubulaires du corps.

Suivant une forme particulière de l'invention, au moins à une de ses extrémités, le corps tubulaire formé de fils multiples tressés présente, à l'état de repos, un évasement où les fils multiples suivent une spirale spatiale à rayons croissants. Une telle forme de réalisation est particulièrement intéressante pour le traitement par exemple d'anévrismes aortiques, où il se produit un rétrécissement du collet de l'anévrisme proximal ou distal. Le tuteur suivant l'invention, en particulier son extrémité évasée, présente une résistance à l'écrasement par la paroi artérielle du fait du rétrécissement du collet. La détente radiale de la partie évasée du tuteur favorise en outre l'absence de migration le long de la paroi de la cavité où il doit être appliqué.

Il est prévu, suivant une forme très avantageuse de l'invention, un évasement à l'extrémité du tuteur dont les génératrices se terminent en hyperbole. Comme on le verra par la suite, cette forme de réalisation présente le grand avantage d'une très grande simplicité de fabrication et d'une reproductibilité parfaite de l'angle de tressage.

Il est aussi prévu, suivant la présente invention, que le corps tubulaire du tuteur comporte, à l'état de repos, deux extrémités évasées de grand diamètre et, entre celles-ci, une section transversale de corps tubulaire de petit diamètre et que, depuis chacune des extrémités évasées jusqu'à la section de petit diamètre, le corps tubulaire présente en section transversale un diamètre diminuant de manière continue. Par cette forme de réalisation, le tuteur s'oppose à tout ballonnement à l'intérieur d'un anévrisme, tout en conservant ses bonnes propriétés d'accrochage par ses extrémités évasées, lors du largage dans une cavité tubulaire d'un corps humain ou animal. Et cela, même si le chirurgien a réduit par découpage une partie de la longueur du tuteur. La nouvelle extrémité formée reste encore évasée par rapport au reste du tuteur. Enfin, la détente radiale des parties évasées du tuteur reste maintenue lors du largage, et les parties évasées, en épousant parfaitement les formes des parois vasculaires où elles sont larguées empêchent de manière efficace une migration du tuteur le long de ces parois.

Il est prévu, suivant une forme avantageuse de l'invention, que, entre au moins l'une des extrémités évasées et la section transversale de petit diamètre, le corps tubulaire comporte une genératrice en forme de droite. Suivant une forme de réalisation préférée de l'invention, entre au moins l'une des extrémités évasées et la section transversale de petit diamètre, le corps tubulaire comporte une génératrice en forme de demi-hyperbole. Dans ce cas, le tuteur a une forme d'hyperboloïde. Suivant une autre forme de réalisation de l'invention, entre au moins l'une des extrémités évasées et la section transversale de petit diamètre, le corps tubulaire comporte une génératrice en forme de segment de cercle.

A l'état de repos, le corps tubulaire comporte avantageusement des fils multiples orientés sous un angle égal, de préférence inférieur, à 45° par rapport à une de ses génératrices. Suivant l'invention, l'angle de tressage n'a pas besoin d'être élevé, pour obtenir de bonnes propriétés de stabilité dimensionnelle. Par conséquent, il s'ensuit une amélioration notable du largage du tuteur dans la cavité où il doit être appliqué.

Suivant une autre forme avantageuse de réalisation de l'invention, le corps tubulaire présente une surface de paroi externe et une surface de paroi interne et il comporte sur au moins une de ces deux surfaces de paroi un recouvrement expansible. Un tel recouvrement peut être appliqué sur le tuteur selon n'importe quelle technique connue, par exemple conformément à l'enseignement de la EP-A-0603959. Cela permet de former par exemple des endoprothèses luminales de 40 mm de diamètre au repos, dont le recouvrement est réalisé en fibres de polycarbonate-uréthanne ou autres d'un diamètre de fibre de 10-20 µm. Le diamètre de telles endoprothèses peut alors être réduit à un diamètre de 4 à 5 mm pour l'insertion dans un introducteur. Il est bien entendu que le recouvrement peut être d'une nature différente et qu'il peut être réalisé selon d'autres techniques bien connues, sans sortir du cadre de l'invention.

L'invention a également pour objet un dispositif médical à introduire dans une cavité d'un corps humain ou animal, qui comprend un tuteur auto-expansible suivant l'invention.

Il est également prévu suivant l'invention un procédé de préparation d'un tuteur auto-expansible pour dispositif médical à introduire dans une cavité d'un corps selon la rev. 13.

On connaît depuis longtemps dans le domaine de la câblerie la fabrication de tresses tubulaires formées de fils métalliques. Les problèmes à résoudre dans ce domaine sont toutefois totalement différents et les exigences posées aux tresses dans la câblerie sont tout à fait distinctes. Un procédé de tressage de tuteur auto-expansible est évoqué dans le US-A-5061275.

Suivant l'invention on prévoit que le procédé décrit ci-dessus comprend, préalablement au tressage, un envidage d'au moins un fuseau par des premiers fils susdits disposés côte à côte au nombre d'au moins deux pour former un premier fil multiple et un envidage d'au moins un fuseau par des deuxièmes fils susdits disposés côte à côte au nombre d'au moins deux pour former un deuxième fil multiple, et que, pendant l'enroulement, tous les premiers fils flexibles de chaque premier fil multiple croisent tous les deuxièmes fils rigides flexibles de chaque deuxième fil multiple croisé, du même côté du deuxième fil multiple croisé, et tous les deuxièmes fils flexibles de chaque deuxième fil multiple croisent tous les premiers fils rigides flexibles de chaque premier fil multiple croisé, du même côté du premier fil multiple croisé.

Un tel procédé permet la fabrication d'un tuteur beaucoup plus performant que ceux de la technique antérieure, sans devoir modifier l'appareillage existant, et donc sans investissement supplémentaire. Il ne comprend pas non plus d'étape préalable de fabrication d'un multifil, tel qu'un toron par exemple.

Suivant un mode de réalisation particulier de l'invention, le procédé comprend un tressage du corps tubulaire à un diamètre supérieur au deuxième diamètre précité, une expansion axiale du corps tubulaire tressé, notamment par son introduction dans un tube à forme interne prédéterminée, et un traitement thermique de durcissement du corps tubulaire ainsi axialement expansé pour le fixer de façon que, après ce traitement, il soit à l'état de repos lorsqu'il présente une forme correspondant à celle de la cavité interne du tube. Ce mode de réalisation permet d'obtenir des angles entre les fils et une génératrice du tuteur parfaitement reproductibles après l'étape de fixation du corps tubulaire dans sa forme correspondant à l'état de repos. Le tube peut avoir une forme quelconque appropriée, à cavité par exemple cylindrique ou d'hyperboloïde.

Suivant un mode perfectionné de réalisation de l'invention, après l'introduction du corps tubulaire tressé dans ledit tube, au moins une de ses extrémités dépasse du tube, en s'évasant. Ainsi, après le traitement thermique de durcissement précité, le tuteur peut être fixé, à l'état de repos, avec un évasement à au moins une de ses extrémités. Dans ce mode de réalisation, l'évasement en question peut prendre automatiquement une forme telle qu'il comporte une génératrice en forme de segment d'hyperbole.

Suivant encore un mode de réalisation de l'invention, le procédé comprend, après le tressage, un enfilage du corps tubulaire tressé autour d'un mandrin de forme appropriée, une tension aux extrémités du corps tubulaire pour l'appliquer sur le mandrin, un traitement thermique de durcissement du corps tubulaire ainsi axialement tendu pour le fixer de façon que, après ce traitement, il présente à l'état de repos la forme du mandrin.

D'autres détails et particularités de l'invention sont indiqués dans les revendications annexées.

L'invention va à présent être décrite de manière plus détaillée dans la description qui suit de formes de réalisation non limitatives et avec référence aux dessins annexés.

La figure 1 représente une vue latérale d'une partie d'extrémité d'une forme de réalisation de tuteur auto-expansible suivant l'invention.

La figure 1A représente, à l'échelle agrandie, un détail du tressage des fils multiples du tuteur de la figure 1.

La figure 2 représente une vue latérale d'une partie d'extrémité d'une deuxième forme de réalisation de tuteur suivant l'invention.

La figure 3 représente une vue latérale d'une partie d'extrémité d'une troisième forme de réalisation de tuteur suivant l'invention.

La figure 4 représente une vue latérale d'une quatrième forme de réalisation de tuteur auto-expansible suivant l'invention.

La figure 5 représente une vue latérale d'une cinquième forme de réalisation de tuteur suivant l'invention.

La figure 6 représente une vue latérale d'une sixième forme de réalisation de tuteur suivant l'invention.

La figure 7 représente un schéma général de l'appareillage de tressage et la figure 8 une vue plus détaillée de l'opération de tressage.

La figure 9 représente une vue partielle d'un tuteur suivant l'invention en fin de fabrication.

Sur les différentes figures, les éléments identiques ou analogues portent les mêmes références.

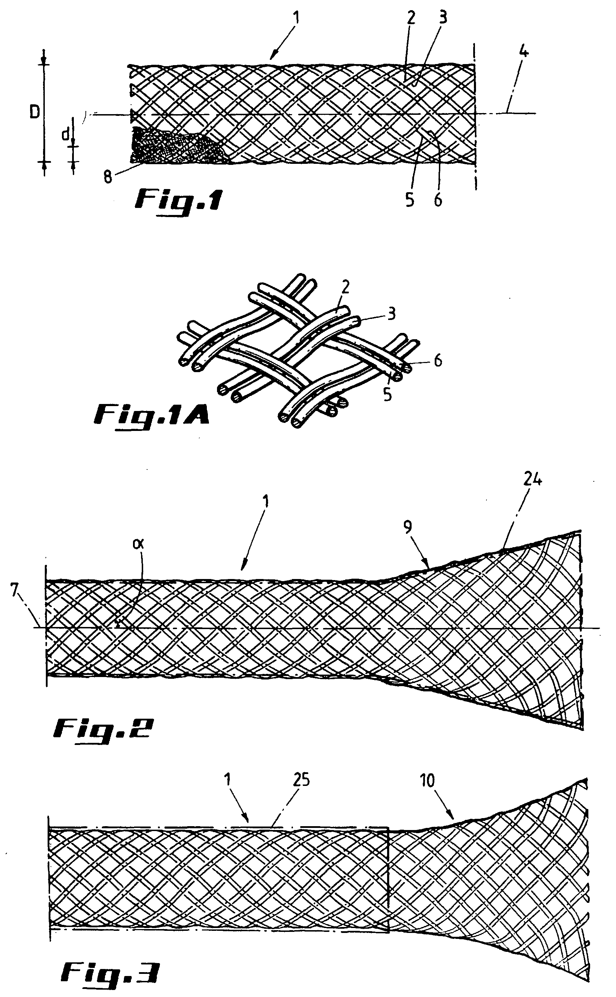

Ainsi qu'il ressort de la figure 1 le tuteur auto-expansible suivant l'invention, illustré partiellement, est formé d'un corps tubulaire 1 présentant à l'extrémité illustrée une forme cylindrique. Le corps tubulaire 1 est formé de fils tressés. Il comprend des premiers fils rigides flexibles, par exemple 2 et 3, qui sont disposés côte à côte, ici au nombre de deux, et qui forment ainsi des premiers fils multiples enroulés suivant un premier sens hélicoïdal autour de l'axe longitudinal 4 du corps tubulaire.

Il comprend aussi des deuxièmes fils rigides 5 et 6 qui sont disposés côte à côte, ici au nombre de deux, et qui forment ainsi des deuxièmes fils multiples enroulés suivant un deuxième sens hélicoïdal, inverse au premier. Il est évident qu'on peut prévoir trois, quatre ou davantage de fils disposés côte à côte pour former un fil multiple suivant l'invention (voir par exemple figure 5). Il faut aussi noter que les fils disposés côte à côte pour former un fil multiple sont contigus sur quasiment la totalité de leur longueur. C'est uniquement pour des raisons de clarté et de lisibilité des figures que les fils formant un fil multiple, comme les fils 2 et 3 ou respectivement 5 et 6, sont représentés légèrement écartés l'un de l'autre.

Comme on peut le voir sur les figures 1 à 3, les fils multiples du corps tubulaire 1 se croisent suivant une disposition tressée, le mode de tressage pouvant être variable comme on l'a déjà indiqué précédemment. Il ressort en particulier de la figure 1A, que tous les fils flexibles formant un même fil multiple au sens de l'invention suivent un parcours rigoureusement parallèle dans la tresse.

Les fils multiples utilisés sont constitués de toute matière qui est appropriée pour l'introduction dans le corps humain et animal et qui présente une rigidité et une flexibilité suffisantes pour la réalisation d'un tuteur auto-expansible. On peut par exemple prévoir des fils métalliques inoxydables, biocompatibles. De tels fils répondant aux normes ISO 5832/7 et/ou ASTM F1058-91 sont hautement souhaitables. On peut par exemple citer des fils PHYNOX à désignation AFNOR : K13 C20 N16 Fe15 Do7, mis sur le marché par la firme Sprint Metal, Paris, France. Il est bien entendu que d'autres fils métalliques ou des fils en d'autres matières, par exemple en matières plastiques à mémoire élastique peuvent être utilisés.

Sur la figure 1, le corps tubulaire est représenté à l'état de repos. Dans cet état il présente, dans cet exemple de réalisation, un diamètre D d'environ 28 mm. Sa longueur est à choisir en fonction de l'utilisation du tuteur. Dans son état de travail, c'est-à-dire au moment où il devra être introduit dans un introducteur connu, non représenté, le tuteur devra présenter un diamètre d qui peut atteindre 3 à 4 mm. L'état de travail est obtenu par compression radiale sur le tuteur et/ou par écartement des extrémités du tuteur en direction axiale. A l'état de travail, le tuteur présente donc une longueur supérieure à sa longueur à l'état de repos. L'agencement des fils multiples suivant l'invention n'entrave en aucune manière l'obtention de cet état de travail du tuteur.

A cet état de repos, les fils présentent, dans les formes de réalisation illustrées, par rapport à une génératrice (7 sur la figure 2) du tuteur un angle au maximum de 45°, de préférence inférieur à celui-ci. Cela offre le grand avantage que le tuteur, à l'état de travail, inséré dans son introducteur, n'a pas une longueur excessive avec tous les inconvénients de largage que cela soulève, comme déjà indiqué précédemment. Un tel angle de 45° n'est toutefois pas critique pour l'invention et peut selon les circonstances être dépassé.

Comme on peut le voir sur la figure 1, le corps tubulaire 1 peut être revêtu sur sa surface interne d'un recouvrement 8 partiellement représenté. Ce recouvrement peut être en n'importe quelle matière biocompatible appropriée, notamment pour la fabrication d'endoprothèses luminales (voir EP-A-0603959). Un recouvrement sur la surface externe peut être prévu au lieu du recouvrement interne 8 ou simultanément à celui-ci. L'utilisation de fils multiples suivant l'invention offre en outre l'avantage d'une relativement grande surface de contact pour avoir une meilleure adhésion avec un tel recouvrement.

Sur la figure 2, est illustré un tuteur suivant l'invention dont le corps tubulaire 1 présente à une extrémité un évasement 9. Comme on peut le voir, à cet endroit, les fils multiples suivent une spirale spatiale à rayons croissants. Dans cet exemple de réalisation, la génératrice 7, à l'endroit de l'évasement 9, forme une droite qui s'écarte progressivement vers l'extérieur de l'axe 4. L'évasement 9 est donc ici de forme tronconique.

Sur la figure 3, est illustré un tuteur dont le corps tubulaire 1 présente à une extrémité un évasement 10 dont la génératrice a une forme de segment d'hyperbole. Cet évasement donne lieu à une extrémité 10 du tuteur 1, en forme d'hyperboloïde tronqué.

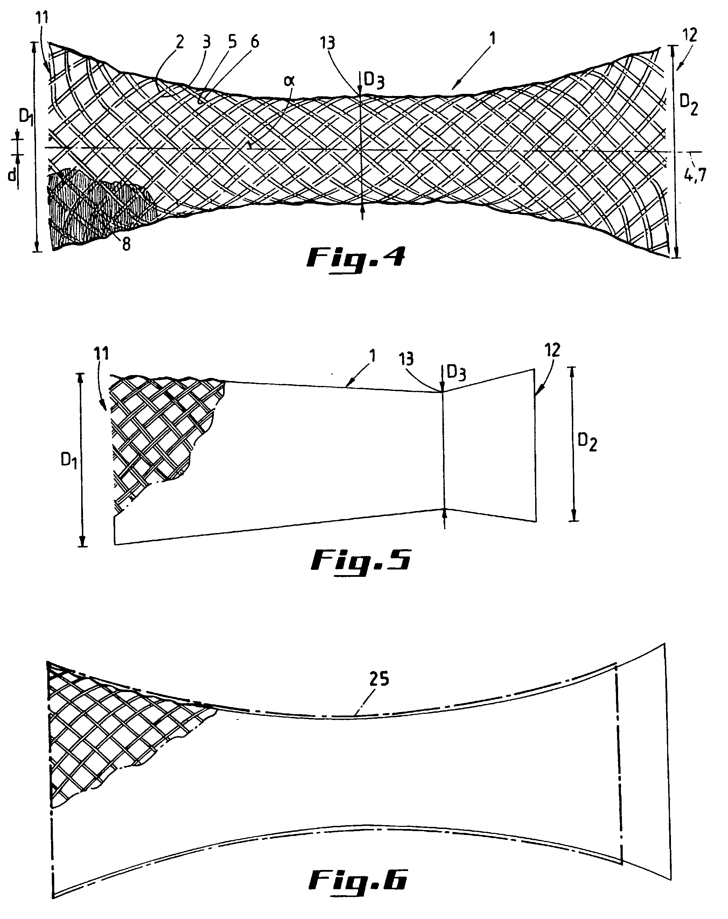

Ainsi qu'il ressort de la figure 4, le tuteur auto-expansible suivant l'invention est formé d'un corps tubulaire 1 présentant dans cet exemple une forme d'hyperboloïde.

Sur la figure 4, le corps tubulaire 1 est représenté à l'état de repos. Dans cet état il présente, dans cet exemple de réalisation, une première extrémité évasée 11 de diamètre D₁ et une deuxième extrémité évasée 12 de diamètre D₂ égal à D₁. A mi-distance de ces deux extrémités évasées est située une section transversale 13 du corps tubulaire dont le diamètre D₃ est inférieur à D₁ et D₂. Le diamètre D₃ peut être par exemple d'environ 28 mm, et les diamètres D₁ et D₂ égaux à par exemple 58 mm. Entre chaque extrémité évasée 11 ou 12 et la section transversale 13 de petit diamètre, le corps tubulaire 1 présente, en section transversale, un diamètre qui diminue de manière continue. Dans le cas illustré cette diminution est telle que le corps tubulaire comporte une génératrice 7 en forme d'hyperbole.

A l'état de repos, les fils présentent, dans cette forme de réalisation illustrée, par rapport à une génératrice 7 du tuteur un angle α qui varie constamment. Dans sa partie centrale, cet angle est de préférence au maximum de 45°, avantageusement inférieur à cette valeur. Aux extrémités évasées il est évidemment beaucoup plus grand. Cela offre le grand avantage que le tuteur, à l'état de travail, inséré dans son introducteur, n'a pas une longueur excessive avec tous les inconvénients de largage que cela soulève, comme déjà indiqué précédemment. Par ailleurs, la détente radiale des extrémités est excellente et permet un bon accrochage du tuteur, en empêchant une migration de celui-ci au cours ou après le largage.

Sur la figure 5, est illustré un tuteur suivant l'invention dont le corps tubulaire 1 présente une première extrémité évasée 11 de diamètre D₁ et une deuxième extrémité 12 de diamètre D₂ inférieur à D₁. La section transversale 13 de petit diamètre D₃ est située ici à une distance plus courte de l'extrémité 12 que de l'extrémité 11, ce qui donne lieu à un corps tubulaire de forme asymétrique. Entre chaque extrémité évasée 11 ou 12, et la section transversale 13 de petit diamètre, le corps tubulaire comporte une génératrice en forme de droite, qui s'écarte progressivement vers l'extérieur de l'axe longitudinal 4, ce qui donne lieu à une forme de diabolo tronconique. Dans cet exemple de réalisation, les fils multiples tressés suivant une hélice à rayons croissants ont été représentés de manière partiellement brisée. Ici chaque fil multiple comprend 3 fils disposés côte à côte.

Sur la figure 6, est illustré un tuteur dont le corps tubulaire 1 comporte entre ses extrémités évasées une génératrice en forme de segment de cercle.

Il est évident qu'on peut prévoir une série d'autres génératrices pour réaliser un tuteur suivant l'invention, par exemple un segment d'ellipse, d'ovale, d'hypocycloïde, de parabole, de caténoïde, et de courbes analogues.

Il doit être entendu que les génératrices de part et d'autre de la section transversale de petit diamètre peuvent être de nature et de valeur différentes.

Les formes de réalisation suivant l'invention illustrées sur les figures 2 à 6 offrent le grand avantage de permettre une bonne fixation du tuteur lors du largage, sans migration ultérieure dans la cavité. L'accrochage à la paroi de celle-ci se fait de manière plus intense à l'extrémité qui est la première larguée, grâce à sa forme évasée, et celle-ci retient le dispositif largué dans la position qu'on lui a imposé. Un tel tuteur suivant l'invention, à deux extrémités évasées, s'avère très avantageux notamment lors de l'introduction d'une endoprothèse pour traiter un anévrisme d'artère abdominale. Ses deux extrémités épousent la forme des collets de l'anévrisme en retenant ainsi parfaitement l'endoprothèse, qui est soumise à cet endroit aux sollicitations de la pression artérielle. La partie centrale du tuteur suivant les figures 4 à 6, cambrée vers l'intérieur et rappelée dans ce sens, résiste parfaitement à cette pression artérielle dans l'anévrisme, là où plus aucune compression radiale n'est excercée sur le tuteur par les parois vasculaires.

Les tuteurs suivant les figures 2 à 6 sont aussi tout à fait appropriés pour le traitement d'anévrismes d'artère sous-clavière, où le tuteur peut se trouver fortement coudé à une extrémité. Au lieu de s'écraser en se refermant à cette extrémité, comme les tuteurs cylindriques de l'art antérieur, le tuteur reste dans une position largement ouverte à son extrémité, grâce à la forme de celle-ci.

Un sectionnement de l' endoprothèse, et donc de son tuteur, par le chirurgien pour une mise à longueur juste avant le traitement ne présente aucun risque, car la forme évasée aux extrémités subsiste malgré cette découpe de dernière minute. Des problèmes de stockage d'endoprothèses de longueurs variables sont ainsi résolus.

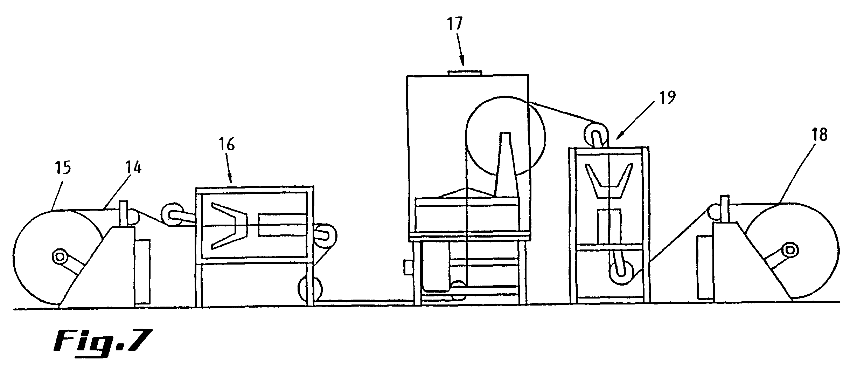

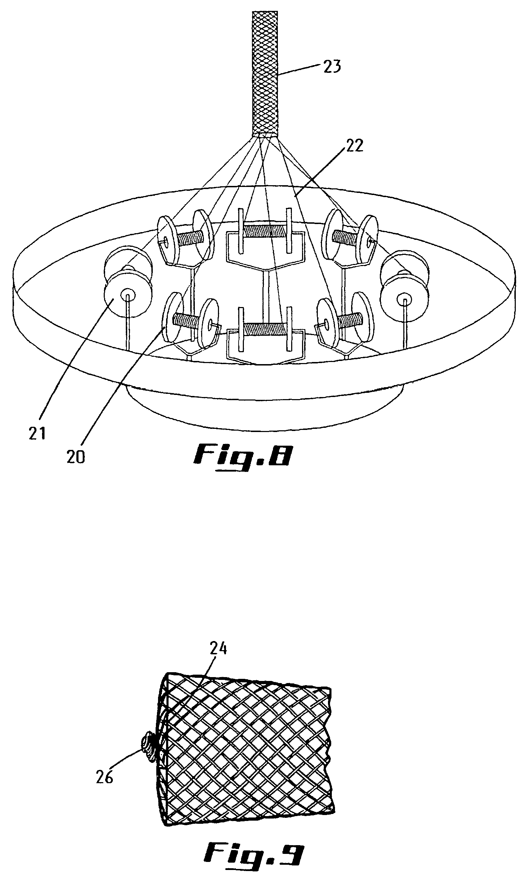

La fabrication d'un corps tubulaire tressé est connue depuis longtemps dans la technique de la câblerie et on fait usage ici de cette technique qui est illustrée sur les figures 7 et 8 annexées. Sur la figure 7, un câble porteur 14 est déroulé depuis une bobine 15. Un système de guidage, désigné d'une manière générale par la référence 16, guide et amène sous tension ce câble 14 qui passe alors dans la machine à tresser 17 schématisée ici. Il sort de cette machine, muni d'un corps tubulaire tressé et il est ainsi enroulé sur une bobine 18 après avoir été amené sous tension par un nouveau système de guidage 19.

La machine à tresser 17 utilisée est représentée de manière un peu plus détaillée sur la figure 8. Comme machine à tresser de ce genre, on peut utiliser par exemple une machine de modèle DBH ou DB mise sur le marché par la firme Spiraltex, Meximieux, France. Les fuseaux d'une telle machine (dont seuls quelques-uns ont été représentés) sont divisés en deux groupes, les fuseaux 20 d'un groupe tournant dans le sens inverse des fuseaux 21 de l'autre groupe, autour de l'axe de la tresse. Le câble porteur 14, ici non représenté, passe verticalement au milieu de la tresse.

Suivant l'invention, et à la différence de l'état de la technique en matière de fabrication de tuteurs auto-expansibles, des fils multiples 22 sont dévidés à partir de chaque ou d'une partie des fuseaux. En fait, préalablement au tressage, plusieurs fils simultanément ont été envidés sur au moins une partie des fuseaux, de préférence sur tous. Comme on l'a déjà dit, ces fils multiples issus des fuseaux sont quasiment jointifs sur toute leur longueur et c'est pourquoi il était impossible de les représenter sur la figure 8 autrement que sous la forme d'un fil.

Le choix du nombre des fuseaux dépend du diamètre de la tresse 23 voulue.

Avantageusement, le corps tubulaire tressé présente au sortir du tressage un diamètre légèrement supérieur au diamètre (par exemple D ou D₃) du tuteur fini, à l'état de repos.

On peut ensuite retirer le corps tubulaire tressé de son câble porteur.

D'une manière courante, on peut alors enfiler le corps tubulaire tressé autour d'un mandrin (par exemple 24, sur la figure 2 ou la figure 9) qui présente la forme voulue, appropriée. Sur la figure 2, ce mandrin a donc la forme d'un cylindre de diamètre par exemple D, portant un tronc de cône à une extrémité. Le corps tubulaire tressé est alors soumis à une tension axiale, à ses extrémités. Il est par exemple, comme sur la figure 9, noué en 26 à une extrémité, puis à l'autre, ce qui le soumet à une tension. Le corps tubulaire épouse alors la forme externe du mandrin 24. Il est ensuite soumis à un traitement thermique de durcissement qui fixe le corps tubulaire aux dimensions du mandrin. Après ce traitement, à l'état de repos, le corps tubulaire a la forme illustrée sur la figure 2 ou respectivement sur la figure 4.

Suivant l'invention, il est également prévu en variante d'allonger le corps tubulaire tressé et de l'introduire dans un tube 25, représenté en traits mixtes sur la figure 3 ou la figure 6, qui présente un diamètre interne égal au diamètre souhaité du tuteur fini. Il est alors soumis au traitement thermique de durcissement. Par exemple on peut imaginer qu'une tresse tubulaire de 35 mm de diamètre soit introduite dans un tube de 28 mm de diamètre interne. Le fil subit un durcissement à 550°C pendant 3 heures, sous atmosphère inerte (argon ou azote avec 5% d'hydrogène) ou sous un vide de 10-5 torr.

A la suite de ce traitement, si la tresse a été complètement introduite dans le tube 25 illustré sur la figure 3, le tuteur fini présente une forme cylindrique comme dans l'exemple de réalisation suivant la figure 1. Si, par contre, une ou les deux extrémités de la tresse dépassent du tube 25 suivant la figure 3, celles-ci vont automatique prendre la forme d'un hyperboloïde tronqué, comme illustré sur cette figure 3.

Le grand avantage de cette opération de durcissement en tube est que les tuteurs finis obtiennent un angle entre les fils multiples et une génératrice qui est, dans les mêmes conditions de traitement, toujours identique et parfaitement reproductible.

Les avantages de l'invention vont à présent être illustrés à l'aide d'exemples comparatifs non limitatifs. La méthode de mesure dans ces exemples consiste à évaluer la résistance à la compression radiale des tuteurs. Une boucle d'un fil métallique de 0,12 mm de diamètre, attachée à une extrémité à un support, est passée autour du tuteur à examiner, approximativement au milieu de celui-ci. Différents poids sont alors suspendus à l'autre extrémité du fil et on mesure ensuite le diamètre au niveau de l'étranglement obtenu pour chaque poids suspendu au fil de mesure.

### Exemple 1

Comparaison entre deux tuteurs qui sont formés d'un même nombre de fils (40 fils simples et respectivement 40 fils doubles) et qui présentent approximativement le même angle entre les fils et une génératrice et le même diamètre à l'état de repos. Les fils des tuteurs sont les mêmes et ont été tressés dans les mêmes conditions.

Le tuteur selon la technique antérieure présente à l'état de repos un diamètre de 32,77 mm et un angle de 52°.

Le tuteur selon l'invention présente à l'état de repos un diamètre de 31,4 mm et un angle de 51°.

Réduction du diamètre (mm) du tuteur en fonction de la masse suspendue au fil de mesure.

| Masse (gr) | Double fil | Simple fil |
|---|---|---|
| 0 | 31,40 | 32,77 |
| 5 | 31,03 | 31,34 |
| 10 | 30,77 | 30,56 |
| 20 | 30,56 | 29,19 |
| 30 | 27,94 | 27,61 |
| 50 | 26,10 | 22,87 |
| 100 | 20,90 | 12,68 |
| 150 | 15,39 | 8,23 |
| 180 | 11,99 | 3,38 |
| 200 | 7,99 | 3,13 |

Il ressort clairement de ces résultats de mesure que, à partir de 50g, c'est-à-dire ce qui correspond aux conditions physiologiques dans les artères, des résistances nettement plus favorables sont obtenues du tuteur en doubles fils.

### Exemple 2

Comparaison entre trois tuteurs qui sont formés d'un même nombre de fils (40 fils simples et respectivement 40 fils doubles) et qui présentent approximativement le même diamètre à l'état de repos. L'angle des fils par rapport à une génératrice est de 66° pour le tuteur selon l'état antérieur de la technique, il est de 49° pour un tuteur X suivant l'invention et de 44° pour un tuteur Y suivant l'invention.

A l'état de repos, les trois tuteurs examinés ont une longueur identique, c'est-à-dire de 10 cm environ. On a introduit ensuite le tuteur selon l'art antérieur et le tuteur Y dans un introducteur Balt du type ID5 (5mm de diamètre interne) d'une longueur de 50 cm. Le tuteur selon l'art antérieur y occupe une longueur de 44 cm, le tuteur Y une longueur de 18 cm.

Réduction du diamètre du tuteur en fonction de la masse suspendue au fil de mesure.

| | **Double fil** | | **Simple fil** |
|---|---|---|---|
| Masse (gr) | Tuteur X Diamètre mesuré (mm) | Tuteur Y Diamètre mesuré (mm) | Diamètre mesuré (mm) |
| 0 | 27,50 | 27,3 | 27,50 |
| 5 | 27,22 | 26,98 | 27,30 |
| 10 | 27,00 | 26,59 | 27,10 |
| 20 | 25,37 | 26,31 | 25,33 |
| 30 | 24,80 | 25,12 | 25,04 |
| 50 | 22,58 | 23,46 | 23,50 |
| 100 | 16,76 | 15,74 | 16,20 |
| 150 | 12,92 | 12,75 | 11,73 |
| 180 | 9,40 | 8,36 | 9,02 |
| 200 | 7,16 | 6,19 | 5,60 |

Il ressort clairement de cet exemple qu'un tuteur à doubles fils présentant un angle de tressage nettement inférieur à celui utilisé dans certains tuteurs selon la technique antérieure offre une résistance à la compression équivalente, sans les problèmes de largage signalés précédemment.

### Exemple 3

Comparaison entre trois tuteurs qui sont formés, l'un (tuteur C) de 72 fils enroulés individuellement comme dans la technique antérieure, et les deux autres de 36 fils doubles suivant l'invention.

Les tuteurs A-C ont un diamètre à l'état de repos de 32 mm. Les fils des tuteurs A et B présentent un angle par rapport à une génératrice de 53,75° et respectivement 53,25°, ceux du tuteur C un angle de 55°.

| Masse (gr) | **Diamètre réduit mesuré (mm)** | | | | | |
|---|---|---|---|---|---|---|
| | A | % (A) | B | % (B) | C | % (C) |
| 0 | 32,86 | 0,000 | 33,49 | 0,000 | 32,99 | 0,000 |
| 10 | 32,10 | 2,307 | 32,23 | 3,762 | 32,41 | 1,752 |
| 20 | 31,14 | 5,229 | 31,43 | 6,151 | 31,25 | 5,269 |
| 30 | 29,76 | 9,428 | 30,93 | 7,644 | 30,47 | 7,633 |
| 50 | 27,56 | 16,124 | 28,91 | 13,676 | 29,24 | 11,362 |
| 70 | 26,12 | 20,506 | 26,65 | 20,424 | 27,17 | 17,637 |
| 100 | 23,54 | 28,358 | 24,63 | 26,456 | 23,98 | 27,307 |
| 130 | 20,27 | 38,310 | 21,29 | 36,429 | 20,05 | 39,220 |
| 150 | 17,58 | 46,497 | 19,61 | 41,445 | 19,43 | 41,100 |
| 170 | 17,02 | 48,201 | 18,45 | 44,909 | 16,37 | 50,376 |
| 200 | 16,18 | 50,758 | 16,77 | 49,925 | 12,59 | 61,835 |

Il ressort clairement de cet exemple qu'on obtient une résistance à la compression équivalente, sinon meilleure, des tuteurs suivant l'invention par rapport au tuteur fabriqué selon la technique antérieure. Il faut noter toutefois que, pour fabriquer le tuteur C, il a fallu faire appel à un appareillage comprenant une machine à tresser à 72 fuseaux, ce qui est exceptionnel (et coûteux) et est beaucoup plus complexe à manipuler. Une telle machine est aussi encombrante, et beaucoup plus bruyante.

Au contraire les tuteurs A et B peuvent être fabriqués sur les mêmes machines à tresser que celles utilisées jusqu'à présent (par exemple à 36 fuseaux).

Il doit être entendu que la présente invention n'est en aucune façon limitée aux formes et modes de réalisation décrits ci-dessus et que bien des modifications peuvent y être apportées sans sortir du cadre des revendications annexées.

## Revendications

1. Tuteur auto-expansible pour dispositif médical à introduire dans une cavité d'un corps humain ou animal, comprenant un corps tubulaire (1) radialement expansible et rétractable entre un premier diamètre (d), correspondant à un état de travail du tuteur, et un deuxième diamètre (D) supérieur au premier, correspondant à un état de repos du tuteur, et axialement expansible et rétractable entre une première longueur, correspondant à l'état de travail, et une deuxième longueur inférieure à la première, correspondant à l'état de repos, ce corps tubulaire (1) comportant des premiers fils rigides flexibles qui sont disposés côte à côte au nombre d'au moins deux en formant des premiers fils multiples (2, 3) enroulés suivant un premier sens hélicoïdal autour d'un axe longitudinal (4) du corps tubulaire, et des deuxièmes fils rigides flexibles qui sont disposés côte à côte au nombre d'au moins deux en formant des deuxièmes fils multiples (5, 6) enroulés suivant un deuxième sens hélicoïdal inverse au premier autour de l'axe longitudinal susdit, chaque fil multiple enroulé dans un desdits sens croisant des fils multiples enroulés dans l'autre sens suivant une disposition tressée,
**caractérisé en ce que** tous les premiers fils rigides flexibles de chaque premier fil multiple suivent un parcours parallèle et croisent tous les deuxièmes fils rigides flexibles de chaque deuxième fil multiple croisé, du même côté du deuxième fil multiple croisé, et **en ce que** tous les deuxièmes fils rigides flexibles de chaque deuxième fil multiple suivent un parcours parallèle et croisent tous les premiers fils rigides flexibles de chaque premier fil multiple croisé, du même côté du premier fil multiple croisé.

2. Tuteur suivant la revendication 1, **caractérisé en ce que** tous les premiers fils susdits forment des fils multiples susdits (2, 3), et **en ce que** tous les deuxièmes fils susdits forment des deuxièmes fils multiples susdits (5, 6).

3. Tuteur suivant l'une des revendications 1 et 2, **caractérisé en ce que** le corps tubulaire (1) comprenant au moins un fil multiple ainsi formé (2, 3; 5, 6) présente au moins partiellement une forme cylindrique.

4. Tuteur suivant l'une des revendications 1 à 3, **caractérisé en ce que**, au moins à une de ses extrémités, le corps tubulaire comprenant au moins un fil multiple ainsi formé (2, 3, 5, 6) présente, à l'état de repos, un évasement (9, 10), où les fils suivent une spirale spatiale à rayons croissants.

5. Tuteur suivant la revendication 4, **caractérisé en ce que** l'évasement susdit comporte une génératrice en forme de droite ou de segment de courbe, de préférence de segment d'hyperbole.

6. Tuteur suivant l'une des revendications 1 et 2, **caractérisé en ce que**, à l'état de repos, le corps tubulaire (1) comporte deux extrémités évasées (11, 12) de grand diamètre (D₁, D₂) et, entre celles-ci, une section transversale (13) du corps tubulaire de petit diamètre (D₃) et **en ce que**, depuis chacune des extrémités évasées (11, 12) jusqu'à la section de petit diamètre (13), le corps tubulaire (1) présente en section transversale un diamètre qui diminue de manière continue.

7. Tuteur suivant la revendication 6, **caractérisé en ce que**, entre au moins l'une des extrémités évasées (11, 12) et la section transversale (13) de petit diamètre, le corps tubulaire (1) comporte une génératrice (7) en forme de droite, ou en forme de segment de courbe, de préférence de segment d'hyperbole.

8. Tuteur suivant l'une quelconque des revendications 1 à 7, **caractérisé en ce que**, à l'état de repos, le corps tubulaire (1) comporte des fils multiples (2, 3, 5, 6) orientés sous un angle (α) égal, de préférence inférieur, à 45° par rapport à une de ses génératrices.

9. Tuteur suivant l'une quelconque des revendications 1 à 8, **caractérisé en ce que** le corps tubulaire (1) présente une surface de paroi externe et une surface de paroi interne et **en ce qu'**il comporte sur au moins une de ces deux surfaces de paroi un recouvrement expansible (8).

10. Tuteur suivant l'une quelconque des revendications 1 à 9, **caractérisé en ce que** les fils rigides flexibles (2, 3, 5, 6) sont constitués de fils métalliques inoxydables, biocompatibles.

11. Dispositif médical à introduire dans une cavité d'un corps humain ou animal, **caractérisé en ce qu'**il comprend un tuteur auto-expansible suivant l'une quelconque des revendications 1 à 10.

12. Dispositif médical suivant la revendication 11, **caractérisé en ce qu'**il consiste en endoprothèses luminales, dilatateurs, cathéters, greffes et éléments analogues à introduire dans des cavités tubulaires du corps.

13. Procédé de préparation d'un tuteur auto-expansible pour dispositif médical à introduire dans une cavité d'un corps, comprenant un tressage de fils provenant de fuseaux intercalés tournant en sens inverse de façon à former un corps tubulaire radialement expansible et rétractable entre un premier diamètre, correspondant à un état de travail du tuteur, et un deuxième diamètre supérieur au premier, correspondant à un état de repos du tuteur, et axialement expansible et rétractable entre une première longueur, correspondant à l'état de travail, et une deuxième longueur inférieure à la première, correspondant à l'état de repos, le tressage comprenant un enroulement de premiers fils rigides flexibles qui sont disposés côte à côte au nombre d'au moins deux en formant des premiers fils multiples (2, 3) enroulés suivant un premier sens hélicoïdal autour d'un axe longitudinal (4) du corps tubulaire, et un enroulement de deuxièmes fils rigides flexibles qui sont disposés côte à côte au nombre d'au moins deux en formant des deuxièmes fils multiples (5, 6) enroulés suivant un deuxième sens hélicoïdal inverse au premier autour de l'axe longitudinal susdit, et un croisement de chaque fil multiple enroulé dans un desdits sens avec des fils multiples enroulés dans l'autre sens,
**caractérisé en ce qu'**il comprend, préalablement au tressage, un envidage d'au moins un fuseau par des premiers fils susdits disposés côte à côte au nombre d'au moins deux pour former un premier fil multiple et un envidage d'au moins un fuseau par des deuxièmes fils susdits disposés côte à côte au nombre d'au moins deux pour former un deuxième fil multiple, et **en ce que**, pendant l'enroulement, tous les premiers fils flexibles de chaque premier fil multiple croisent tous les deuxièmes fils rigides flexibles de chaque deuxième fil multiple croisé, du même côté du deuxième fil multiple croisé, et tous les deuxièmes fils flexibles de chaque deuxième fil multiple croisent tous les premiers fils rigides flexibles de chaque premier fil multiple croisé, du même côté du premier fil multiple croisé.

14. Procédé suivant la revendication 13, **caractérisé en ce qu'**il comprend un tressage du corps tubulaire à un diamètre supérieur au deuxième diamètre précité, une expansion axiale du corps tubulaire tressé pour qu'il atteigne ledit deuxième diamètre et un traitement fixant le corps tubulaire ainsi expansé axialement de façon qu'après ce traitement il soit à l'état de repos lorsqu'il présente ce deuxième diamètre.

15. Procédé suivant la revendication 14, **caractérisé en ce qu'**il comprend un tressage du corps tubulaire à un diamètre supérieur au deuxième diamètre précité, une expansion axiale du corps tubulaire tressé pour son introduction dans un tube à cavité interne de forme prédéterminée, et un traitement thermique de durcissement du corps tubulaire ainsi axialement expansé pour le fixer de façon que, après ce traitement, il soit à l'état de repos lorsqu'il présente une forme correspondant à celle de la cavité interne du tube.

16. Procédé suivant la revendication 15, **caractérisé en ce que**, après l'introduction du corps tubulaire tressé dans ledit tube, au moins une de ses extrémités dépasse en s'évasant.

17. Procédé suivant la revendication 14, **caractérisé en ce que**, après le tressage, il comprend un enfilage du corps tubulaire tressé autour d'un mandrin de forme appropriée, une tension aux extrémités du corps tubulaire pour l'appliquer sur le mandrin, un traitement thermique de durcissement du corps tubulaire ainsi axialement tendu pour le fixer de façon que, après ce traitement, il présente à l'état de repos la forme du mandrin.

## Patentansprüche

1. Selbstexpandierender Stent zur Einführung einer medizinischen Vorrichtung in eine Körperhöhle eines Menschen oder eines Tiers, umfassend einen röhrenförmigen Körper (1), der radial expandierbar und zwischen einem ersten Durchmesser (d), der einem Arbeitszustand des Stents entspricht, und einem zweiten Durchmesser (D), der größer als der erste ist und einem Ruhezustand des Stents entspricht, einziehbar und axial expandierbar und zwischen einer ersten Länge, die dem Arbeitszustand entspricht, und einer zweiten Länge, die geringer als die erste ist und dem Ruhezustand entspricht, einziehbar ist, wobei dieser röhrenförmige Körper (1) erste feste flexible Fäden, die Seite an Seite in der Zahl von mindestens zwei angeordnet sind, indem sie erste Mehrfachfäden (2, 3) bilden, die in einer ersten Spiralrichtung um eine Längsachse (4) des röhrenförmigen Körpers gewickelt sind, und zweite feste flexible Fäden umfasst, die Seite an Seite in der Zahl von mindestens zwei angeordnet sind, indem sie zweite Mehrfachfäden (5, 6) bilden, die in eine zweite Spiralrichtung, die zur ersten entgegengesetzt ist, um die genannte Längsachse gewickelt sind, wobei jeder in eine der Richtungen gewickelte Mehrfachfaden in die andere Richtung gewickelte Mehrfachfäden in einer geflochtenen Anordnung kreuzt,
**dadurch gekennzeichnet, dass** alle ersten festen flexiblen Fäden jedes ersten Mehrfachfadens einem parallelen Weg folgen und alle zweiten festen flexiblen Fäden jedes zweiten gekreuzten Mehrfachfadens auf derselben Seite des zweiten gekreuzten Mehrfachfadens kreuzen, und dass alle zweiten festen flexiblen Fäden jedes zweiten Mehrfachfadens einem parallelen Weg folgen und alle ersten festen flexiblen Fäden jedes ersten gekreuzten Mehrfachfadens auf derselben Seite des ersten gekreuzten Mehrfachfadens kreuzen.

2. Stent nach Anspruch 1, **dadurch gekennzeichnet, dass** alle genannten ersten Fäden genannte erste Mehrfachfäden (2, 3) bilden, und dass alle genannten zweiten Fäden genannte zweite Mehrfachfäden (5, 6) bilden.

3. Stent nach einem der Ansprüche 1 und 2, **dadurch gekennzeichnet, dass** der röhrenförmige Körper (1), der mindestens einen auf diese Weise gebildeten Mehrfachfaden (2, 3; 5, 6) umfasst, mindestens teilweise eine zylindrische Form aufweist.

4. Stent nach einem der Ansprüche 1 bis 3, **dadurch gekennzeichnet, dass** mindestens an einem seiner Enden der röhrenförmige Körper, der mindestens einen auf diese Weise gebildeten Mehrfachfaden (2, 3, 5, 6) umfasst, im Ruhezustand eine Erweiterung (9, 10) aufweist, in der die Fäden einer Raumspirale mit kreuzenden Radien folgen.

5. Stent nach Anspruch 4, **dadurch gekennzeichnet, dass** die genannte Erweiterung eine Mantellinie in Form einer Geraden oder eines Kurvensegments, vorzugsweise in Form eines Hyperbelsegments umfasst.

6. Stent nach einem der Ansprüche 1 und 2, **dadurch gekennzeichnet, dass** im Ruhezustand der röhrenförmige Körper (1) zwei erweiterte Enden (11, 12) mit großem Durchmesser (D₁, D₂) und zwischen diesen einen Querschnitt (13) des röhrenförmigen Körpers mit kleinem Durchmesser (D₃) umfasst, und dass von jedem der erweiterten Enden (11, 12) bis zum Querschnitt mit kleinem Durchmesser (13) der röhrenförmige Körper (1) im Querschnitt einen Durchmesser aufweist, der kontinuierlich kleiner wird.

7. Stent nach Anspruch 6, **dadurch gekennzeichnet, dass** zwischen mindestens einem der erweiterten Enden (11, 12) und dem Querschnitt (13) mit kleinem Durchmesser der röhrenförmige Körper (1) eine Mantellinie (7) in Form einer Geraden oder in Form eines Kurvensegments, vorzugsweise in Form eines Hyperbelsegments umfasst.

8. Stent nach einem der Ansprüche 1 bis 7, **dadurch gekennzeichnet, dass** im Ruhezustand der röhrenförmige Körper (1) Mehrfachfäden (2, 3, 5, 6) umfasst, die unter einem Winkel (α) gleich, vorzugsweise kleiner als 45° in Bezug auf eine ihrer Mantellinien ausgerichtet sind.

9. Stent nach einem der Ansprüche 1 bis 8, **dadurch gekennzeichnet, dass** der röhrenförmige Körper (1) eine Außenwandfläche und eine Innenwandfläche aufweist, und dass er auf mindestens einer dieser beiden Wandflächen eine expandierbare Abdeckung (8) umfasst.

10. Stent nach einem der Ansprüche 1 bis 9, **dadurch gekennzeichnet, dass** die festen flexiblen Fäden (2, 3, 5, 6) aus rostfreien, biokompatiblen metallischen Fäden gebildet sind.

11. Medizinische Vorrichtung, die in eine Körperhöhle eines Menschen oder eines Tiers eingeführt werden soll, **dadurch gekennzeichnet, dass** sie einen selbstexpandierbaren Stent nach einem der Ansprüche 1 bis 10 umfasst.

12. Medizinische Vorrichtung nach Anspruch 11, **dadurch gekennzeichnet, dass** sie in Luminalendoprothesen, Dilatatoren, Kathetern, Transplantaten und ähnlichen Elementen besteht, die in röhrenförmige Körperhöhlen eingeführt werden sollen.

13. Verfahren zur Herstellung eines selbstexpandierenden Stents zur Einführung einer medizinischen Vorrichtung in eine Körperhöhle, umfassend eine Verflechtung von Fäden, die aus verwundenen Wickeln stammen, die sich in entgegengesetzte Richtung drehen, um einen röhrenförmigen Körper zu bilden, der radial expandierbar und zwischen einem ersten Durchmesser, der einem Arbeitszustand des Stents entspricht, und einem zweiten Durchmesser, der größer als der erste ist und einem Ruhezustand des Stents entspricht, einziehbar und axial expandierbar und zwischen einer ersten Länge, die dem Arbeitszustand entspricht, und einer zweiten Länge, die geringer als die erste ist und dem Ruhezustand entspricht, einziehbar ist, wobei die Verflechtung eine Wicklung von ersten festen flexiblen Fäden, die Seite an Seite in der Zahl von mindestens zwei angeordnet sind, indem sie erste Mehrfachfäden (2, 3) bilden, die in einer ersten Spiralrichtung um eine Längsachse (4) des röhrenförmigen Körpers gewickelt sind, und von zweiten festen flexiblen Fäden, die Seite an Seite in der Zahl von mindestens zwei angeordnet sind, indem sie zweite Mehrfachfäden (5, 6) bilden, die in eine zweite Spiralrichtung, die zur ersten entgegengesetzt ist, um die genannte Längsachse gewickelt sind, und eine Kreuzung jedes in eine der Richtungen gewickelten Mehrfachfadens mit in die andere Richtung gewickelten Mehrfachfäden umfasst,
**dadurch gekennzeichnet, dass** es vor der Verflechtung ein Umwickeln von mindestens einer Spindel mit genannten ersten Fäden, die Seite an Seite in der Zahl von mindestens zwei angeordnet sind, um einen ersten Mehrfachfaden zu bilden, und ein Umwickeln von mindestens einer Spindel mit genannten zweiten Fäden umfasst, die Seite an Seite in der Zahl von mindestens zwei angeordnet sind, um einen zweiten Mehrfachfaden zu bilden, und dass während des Wickelns alle ersten flexiblen Fäden jedes ersten Mehrfachfadens alle zweiten festen flexiblen Fäden jedes zweiten gekreuzten Mehrfachfadens auf derselben Seite des zweiten gekreuzten Mehrfachfadens kreuzen, und dass alle zweiten flexiblen Fäden jedes zweiten Mehrfachfadens alle ersten festen flexiblen Fäden jedes ersten gekreuzten Mehrfachfadens auf derselben Seite des ersten gekreuzten kreuzen.

14. Verfahren nach Anspruch 13, **dadurch gekennzeichnet, dass** es eine Verflechtung des röhrenförmigen Körpers mit einem größeren Durchmesser als der zweite vorgenannte Durchmesser, eine Axialexpansion des geflochtenen röhrenförmigen Körpers, damit er den zweiten Durchmesser erreicht, und eine Behandlung umfasst, die den auf diese Weise axial expandierten röhrenförmigen Körper derart fixiert, dass er sich nach dieser Behandlung im Ruhezustand befindet, wenn er diesen zweiten Durchmesser aufweist.

15. Verfahren nach Anspruch 14, **dadurch gekennzeichnet, dass** es eine Verflechtung des röhrenförmigen Körpers mit einem größeren Durchmesser als der vorgenannte zweite Durchmesser, eine Axialexpansion des geflochtenen röhrenförmigen Körpers für dessen Einführung in ein Rohr mit Innenhöhle mit vorbestimmter Form und eine Wärmebehandlung zur Härtung des auf diese Weise axial expandierten röhrenförmigen Körpers umfasst, um ihn derart zu fixieren, dass er sich nach dieser Behandlung im Ruhezustand befindet, wenn er eine Form aufweist, die jener der Innenhöhle des Rohrs entspricht.

16. Verfahren nach Anspruch 15, **dadurch gekennzeichnet, dass** nach der Einführung des geflochtenen röhrenförmigen Körpers in das Rohr mindestens eines seiner Enden überragt, indem es sich erweitert.

17. Verfahren nach Anspruch 14, **dadurch gekennzeichnet, dass** er nach der Verflechtung ein Wickeln des geflochtenen röhrenförmigen Körpers um einen Dorn geeigneter Form, ein Spannen des röhrenförmigen Körpers an den Enden, um ihn an den Dorn anzulegen, eine Wärmebehandlung zur Härtung des auf diese Weise axial gespannten röhrenförmigen Körpers umfasst, um ihn derart zu fixieren, dass er nach dieser Behandlung im Ruhezustand die Form des Dorns aufweist.

## Claims

1. Autoexpansible stent for a medical device to be introduced into a cavity of a human or animal body, comprising a tubular body (1) which is radially expansible and retractable between a first diameter (d), corresponding to a working state of the stent, and a second diameter (D), greater than the first, corresponding to a resting state of the stent, and is axially expansible and retractable between a first length, corresponding to the working state, and a second length, less than the first, corresponding to the resting state, this tubular body (1) comprising first flexible rigid filaments which are arranged side by side in a number of at least two while forming first multiple filaments (2, 3) wound along a first helicoid direction around a longitudinal axis (4) of the tubular body (1), and second flexible rigid filaments which arranged side by side in a number of at least two while forming second multiple filaments (5, 6) wound along a second helicoid direction opposite to the first around the abovementioned longitudinal axis, each multiple filament wound in one of the said directions crossing filaments wound in the other direction according to a plaited arrangement, **characterized in that** all the first flexible rigid filaments of each first multiple filament follow a parallel path and cross all the second flexible rigid filaments of each second multiple filament, on the same side of the crossed second multiple filament and **in that** all the second flexible rigid filaments of each second multiple filament follow a parallel path and cross all the first flexible rigid filaments of each first multiple filament, on the same side of the crossed first multiple filament.

2. Stent according to Claim 1, **characterized in that** all the abovementioned first filaments form abovementioned multiple filaments (2, 3), and **in that** all the abovementiond second filaments form second abovementioned multiple filaments (5, 6).

3. Stent according to one of Claims 1 and 2, **characterized in that** the tubular body (1) comprising at least one multiple filament thus formed (2, 3; 5, 6) has at least partially a cylindrical shape.

4. Stent according to one of Claims 1 to 3, **characterized in that**, at least at one of its ends, the tubular body comprising at least one multiple filament (2, 3, 5, 6) thus formed has, in the resting state, a flaring (9, 10) in which the filaments follow a spatial spiral with increasing radii.

5. Stent according to Claim 4, **characterized in that** the abovementioned flaring includes a generatrix in the shape of a straight line or of a curve segment, preferably a hyperbola segment.

6. Stent according to one of Claims 1 and 2, **characterized in that**, in the resting state, the tubular body (1) includes two flared ends (11, 12) of large diameter (D₁, D₂) and, between them, a cross-section (13) of the tubular body of small diameter (D₃), and **in that**, from each of the flared ends (11, 12) to the small-diameter cross-section (13), the tubular body (1) has, in cross-section, a diameter which decreases continuously.

7. Stent according to Claim 6, **characterized in that**, between at least one of the flared ends (11, 12) and the small-diameter cross-section (13), the tubular body (1) includes a generatrix (7) in the form of a straight line, or in the form of a curve segment, preferably a hyperbola segment.

8. Stent according to any one of Claims 1 to 7, **characterized in that**, in the resting state, the tubular body (1) includes multiple filaments (2, 3, 5, 6) oriented under an angle (α) equal to, preferably less than, 45° with respect to one of its generatrices.

9. Stent according to any one of Claims 1 to 8, **characterized in that** the tubular body (1) has an external wall surface and an internal wall surface and **in that** it includes, over at least one of these two wall surfaces, an expansible covering (8).

10. Stent according to any one of Claims 1 to 9, **characterized in that** the flexible rigid filaments (2, 3, 5, 6) consist of biocompatible stainless metallic filaments.

11. Medical device to be introduced into a cavity of a human or animal body, **characterized in that** it comprises an autoexpansible stent according to any one of Claims 1 to 10.

12. Medical device according to Claim 11, **characterized in that** it consists of luminal endoprostheses, dilators, catheters, grafts and similar elements to be introduced into tubular cavities of the body.

13. Method for preparing an autoexpansible stent for a medical device to be introduced into a cavity of a body, comprising plaiting filaments originating from interposed reels rotating in the opposite direction so as to form a tubular body which is radially expansible and retractable between a first diameter, corresponding to a working state of the stent, and a second diameter, greater than the first, corresponding to a resting state of the stent, and is axially expansible and retractable between a first length, corresponding to the working state, and a second length, less than the first, corresponding to the resting state, the plaited structure comprising a winding of first flexible rigid filaments which are arranged side by side in a numbering of at least two, while forming first multiple filaments (2, 3) wound along a first helicoid direction around a longitudinal axis (4) of the tubular body and a winding a second flexible rigid filaments which are arranged side by side in a numbering of at least two, while forming second multiple filaments (5, 6) wound along a second helicoid direction opposite to the first, each multiple filaments wound in one of the said helicoid directions crossing multiple filaments wound in the other helicoid direction,
**characterized in that** it comprises, prior to plaiting, winding at least one reel with abovementioned first filaments arranged side by side in a numbering of at least two in order to form a first multiple filament and winding at least one reel with abovementioned second filaments arranged side by side in a numbering of at least two in oder to form a second multiple filament and **in that**, during winding, all the first flexible rigid filaments of each first multiple filament cross all the second flexible rigid filaments of each second multiple filament, on the same side of the crossed second multiple filament, and all the second flexible rigid filaments of each second multiple filament cross all the first flexible rigid filaments of each first multiple filament, on the same side of the crossed first multiple filament.

14. Method according to Claim 13, **characterized in that** it comprises plaiting the tubular body at a diameter greater than the aformentioned second diameter, axial expansion of the plaited tubular body so that it reaches the said second diameter and treatment fixing the tubular body thus expanded axially so that, after this treatment, it is in the resting state when it has this second diameter.

15. Method according to Claim 13, **characterized in that** it comprises plaiting of the tubular body at a diameter greater than the aforementioned second diameter, axial expansion of the plaited tubular body in order to introduce it into a tube having an internal cavity of predetermined shape, and thermal treatment for setting the tubular body thus axially expanded in order to fix it so that, after this treatment, it is in the resting state when it has a shape corresponding to the internal cavity of the tube.

16. Method according to Claim 15, **characterized in that**, after introduction of the plaited tubular body into the said tube, at least one of its ends protrudes while flaring.

17. Method according to Claim 14, **characterized in that**, after plaiting, it comprises threading the plaited tubular body around a mandrel of suitable shape, tensioning at the ends of the tubular body in order to apply it onto the mandrel, thermal treatment for setting the tubular body thus axially tensioned in order to fix it so that, after this treatment, it has, in the resting state, the shape of the mandrel.
